(19) Europäisches Patentamt
European Patent Office
Office européen des brevets

(11) Veröffentlichungsnummer: **0 238 575**
**B1**

(12) # EUROPÄISCHE PATENTSCHRIFT

(45) Veröffentlichungstag der Patentschrift: **02.08.89**

(51) Int. Cl.⁴: **C 07 C 47/58**, C 07 C 45/78, C 07 C 45/82

(21) Anmeldenummer: **86905778.6**

(22) Anmeldetag: **12.09.86**

(86) Internationale Anmeldenummer: **PCT/EP 86/00526**

(87) Internationale Veröffentlichungsnummer: **WO 87/01695 (26.03.87 Gazette 87/07)**

(54) **VERFAHREN ZUR GEWINNUNG VON VANILLIN.**

(30) Priorität: **20.09.85 DE 3533562**

(43) Veröffentlichungstag der Anmeldung: **30.09.87 Patentblatt 87/40**

(45) Bekanntmachung des Hinweises auf die Patenterteilung: **02.08.89 Patentblatt 89/31**

(84) Benannte Vertragsstaaten: **FR GB SE**

(56) Entgegenhaltungen:
**DE-A-1 493 190**
**FR-A-2 578 246**
**US-A-2 069 185**
**US-A-4 021 493**

(73) Patentinhaber: **Fried. Krupp Gesellschaft mit beschränkter Haftung, Altendorfer Strasse 103, D-4300 Essen 1 (DE)**

(72) Erfinder: **COENEN, Hubert, Wortbergrode 13, D-4300 Essen 1 (DE)**
Erfinder: **KONRAD, Reinhard, Rosenbaumweg 28, D-4630 Bochum (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 238 575 B1**

LIBER, STOCKHOLM 1989

**Beschreibung**

**Verfahren zur Gewinnung von Vanillin**

Die Erfindung bezieht sich auf ein Verfahren zur Gewinnung von Vanillin durch Extraktion eines vanillinhaltigen, wasserhaltigen, flüssigen Stoffgemisches mit Kohlendioxid bei einer Temperatur von 0 bis 110°C sowie einem Druck von 30 bis 400 bar und anschließende Abscheidung des Vanillins aus dem vanillinhaltigen Kohlendioxid.

Vanillin (3-Methoxy-4-hydroxy-Benzaldehyd) bildet farblose nadelförmige Kristalle mit einem Schmelzpunkt von 82°C und wird als Aroma- sowie Riechstoff verwendet. Vanillin wird durch chemische Synthese aus Eugenol oder Guajacol hergestellt oder aus den oxidierten Sulfit-Ablaugen des Holzaufschlusses extrahiert. Beim Holzaufschluß, welcher der Zellstoffgewinnung dient, werden Holzstückchen mit einer $NaHSO_3$-, $Mg(HSO_3)_2$-oder $Ca(HSO_3)_2$-Lösung unter Druck gekocht, wobei sich das Lignin löst und mit der Sulfit-Ablauge abgeführt wird. Bei der Oxidation dieser Ablauge mit Nitrobenzol in alkalischem Medium unter Druck oder mit einem sauerstoffhaltigen Gas in Gegenwart eines Oxidationskatalysators bildet sich unter anderem Vanillin, das dann durch n-Butanol oder Toluol extrahiert werden kann.

Aus der DE-AS 1 493 190 ist ein Verfahren zur Auftrennung flüssiger und/oder fester Stoffgemische bekannt, die organische Verbindungen und/oder organische Gruppen aufweisende Verbindungen enthalten. Bei diesem bekannten Verfahren wird das Stoffgemisch mit einem unter überkritischen Bedingungen der Temperatur und des Drucks stehenden Gas im Temperaturbereich bis 100°C über seiner kritischen Temperatur behandelt, und nach Abtrennung der beladenen überkritischen Gasphase werden die in ihr enthaltenen Verbindungen durch Entspannung und/oder Temperaturerhöhung zurückgewonnen. Als gasförmiges Extraktionsmittel kann auch Kohlendioxid verwendet werden. Wird dieses bekannte Verfahren zur Gewinnung von Vanillin aus vanillinhaltigen, flüssigen Stoffgemischen angewendet, so ergibt sich in nachteiliger Weise, daß bei der Abscheidung des Vanillins durch Entspannung und/oder Temperaturerhöhung auch die anderen mitextrahierten organischen Verbindungen teilweise abgeschieden werden, wodurch das Vanillin unvertretbar stark verunreinigt ist. Außerdem kann das bekannte Verfahren nur teilkontinuierlich betrieben werden, was sich nachteilig auf die Wirtschaftlichkeit des Verfahrens auswirkt.

Der Erfindung liegt daher die Aufgabe zugrunde, ein Verfahren zur Gewinnung von Vanillin zu schaffen, das es ermöglicht, Vanillin in weitgehend reiner Form kontinuierlich aus vanillinhaltigen, wasserhaltigen, flüssigen Stoffgemischen durch Extraktion mit Kohlendioxid abzutrennen.

Die der Erfindung zugrundeliegende Aufgabe wird dadurch gelöst, daß das während der Extraktion anfallende vanillinhaltige Kohlendioxid bei der Extraktionstemperatur und dem Extraktionsdruck durch eine wässrige $NaHSO_3$-, $KHSO_3$-, $Na_2SO_3$- oder $K_2SO_3$-Lösung geleitet, daß die vanillinhaltige $NaHSO_3$-, $KHSO_3$-, $Na_2SO_3$- oder $K_2SO_3$-Lösung anschließend mit Schwefelsäure bis zu einem pH-Wert von 2 bis 4 angesäuert und daß das vanillinfreie Kohlendioxid in die Extraktionsstufe zurückgeführt wird. Es hat sich gezeigt, daß das Vanillin von den vorgenannten Hydrogensulfit- oder Sulfit-Lösungen nahezu selektiv aus der vanillinhaltigen Kohlendioxidphase abgetrennt wird und daher in sehr reiner Form anfällt. Das Endprodukt hat einen Vanillingehalt von 90 bis 95 %. Außerdem erfolgt die Abtrennung des Vanillins unter den Extraktionsbedingungen von Druck und Temperatur, wodurch sich überraschende Vorteile ergeben. Da die Extraktion und die Abscheidung des Vanillins unter isothermen und isobaren Bedingungen erfolgen, wird eine kontinuierliche Fahrweise des Verfahrens zur Vanillingewinnung ermöglicht, wodurch das Verfahren erst in der Praxis wirtschaftlich anwendbar ist. Obwohl es an sich bekannt ist, daß Aldehyde in wässriger Phase mit dem Hydrogensulfit-Ion nach der Gleichung

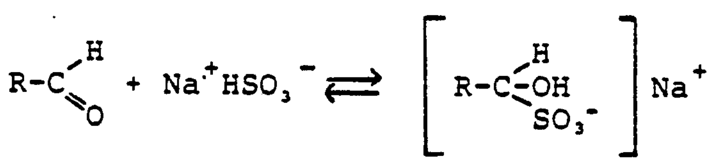

Addukte bilden, die meist als gut kristallisierende und schwer lösliche Natriumsalze ausfallen, war es überraschend, daß das Vanillin aus einer flüssigen oder überkritischen vanillinhaltigen Kohlendioxidphase mit einer $NaHSO_3$-, $KHSO_3$-, $Na_2SO_3$- oder $K_2SO_3$-Lösung weitgehend selektiv und weitgehend quantitativ ausgewaschen werden kann. Beim Einsatz einer $Na_2SO_3$-oder $K_2SO_3$-Lösung wird das Hydrogensulfit-Ion nach der Gleichung

$$Me_2SO_3 + H_2O + CO_2 \leftrightarrows MeHSO_3 + MeHCO_3$$
$$Me = Na, K$$

gebildet.

Das erfindungsgemäße Verfahren kann besonders erfolgreich durchgeführt werden, wenn das vanillinhaltige Kohlendioxid in der wässrigen $NaHSO_3$-, $KHSO_3$-, $Na_2SO_3$- oder $K_2SO_3$ -Lösung eine Verweilzeit von 0,1 bis 3 Minuten hat. Bei dieser Verweilzeit wird das Vanillin nahezu quantitativ in der Hydrogensulfit- oder Sulfit-Lösung adsorbiert, wobei es besonders vorteilhaft ist, daß das Vanillin-Hydrogensulfit-Addukt in Wasser eine gute Löslichkeit hat.

Die Abtrennung des Vanillins aus der Hydrogensulfit- oder Sulfit-Lösung gelingt in kurzer Zeit nahezu quantitativ, wenn die vanillinhaltige $NaHSO_3$-, $KHSO_3$-, $Na_2SO_3$- oder $K_2SO_3$ -Lösung während oder nach dem Ansäuern mit Schwefelsäure auf eine Temperatur von 50 bis 90°C erhitzt wird. Durch das Erwärmen der Lösung während oder nach dem Ansäuern fällt das Vanillin in Form filtrierbarer Kristalle oder kleiner Flüssigkeitströpfchen aus, wobei die Flüssigkeitströpfchen nach entsprechender Abkühlung ebenfalls gut kristallisieren.

Überraschenderweise hat sich gezeigt, daß das erfindungsgemäße Verfahren besonders erfolgreich durchführbar ist, wenn als vanillinhaltiges, wasserhaltiges, flüssiges Stoffgemisch die oxidierte Ablauge des Sulfit-Holzaufschlusses verwendet wird. Auf diese Weise kann das Vanillin, das in den oxidierten Ablaugen der Sulfit-Zellstoffabrikation enthalten ist, mit vertretbaren Kosten in hoher Reinheit gewonnen werden. Außerdem kann das beim erfindungsgemäßen Verfahren anfallende Schwefeldioxid in vorteilhafter Weise zur Herstellung der Lauge verwendet werden, die zum Sulfit-Holzaufschluß benötigt wird.

Der Gegenstand der Erfindung wird nachfolgend anhand der Zeichnung und eines Ausführungsbeispiels näher erläutert.

In der Zeichnung ist das Fließbild des erfindungsgemäßen Verfahrens dargestellt. Im Vorratstank 1 befindet sich die oxidierte Sulfit-Ablauge des Holzaufschlusses, die 0,2 bis 2 % Vanillin enthält. Die oxidierte Sulfit-Ablauge gelangt über die Leitung 2 in die Pumpe 3, wo die Ablauge auf den Extraktionsdruck gebracht wird. Anschließend wird die Sulfit-Ablauge über die Leitung 4 kopfseitig in die Extraktionskolonne 5 geführt, wo sie im Gegenstrom bei einer Temperatur von 0 bis 110°C und einem Druck von 30 bis 400 bar mit Kohlendioxid behandelt wird. Dabei belädt sich das Kohlendioxid mit Vanillin, während die nahezu vanillinfreie Sulfit-Ablauge über die Leitung 11 abgeführt wird. Die vanillinfreie Sulfit-Ablauge wird eingedampft, und der dabei anfallende Rückstand wird verbrannt.

Das vanillinhaltige Kohlendioxid gelangt über die Leitung 12 in den Gaswäscher 13, der mit einer wässrigen Lösung von $NaHSO_3$, $KHSO_3$, $Na_2SO_3$ oder $K_2SO_3$ beschickt wird. Der Gaswäscher 13 wird nach dem Gegenstromprinzip betrieben. Die Hydrogensulfit- oder Sulfit-Lösung entzieht dem vanillinhaltigen Kohlendioxid das Vanillin. Um möglichst geringe Mengenströme bewältigen zu müssen und um Aufkonzentrierungsprobleme zu vermeiden, werden möglichst konzentrierte Hydrogensulfit- oder Sulfit-Lösungen verwndet, die aus dem Vorratsstank 15 über die Leitung 16 in den Gaswäscher 13 gelangen. Der Gaswäscher 13 und die Extraktionskolonne 5 werden bei gleicher Temperatur und gleichem Druck betrieben.

Das vanillinfreie Kohlendioxid gelangt über die Leitung 14 in den Wärmeaustauscher 27, wo es verflüssigt wird. Kohlendioxidverluste werden dadurch ausgeglichen, daß aus dem Vorratstank 25 über die Leitung 26 Kohlendioxid in die Leitung 14 zudosiert wird. Auf die Abtrennung der organischen Verunreinigungen, die in der Extraktionskolonne 5 zusammen mit dem Vanillin extrahiert und im Gaswäscher 13 nicht aus dem Kohlendioxid ausgewaschen werden, kann man verzichten, da das im Kreislauf geführte Kohlendioxid beim zweiten Durchgang durch die Extraktionskolonne 5 keine weiteren Verunreinigungen aufnimmt, denn bei gleichbleibenden Extraktionsbedingungen tritt schnell eine Sättigung des Kohlendioxids mit den organischen Verunreigungen ein. Das verflüssigte Kohlendioxid gelangt über die Leitung 10 in die Pumpe 9, wo die Einstellung des Extraktionsdrucks erfolgt. Anschließend wird das Kohlendioxid über die Leitung 8 zur Einstellung der Extraktionstemperatur in den Wärmeaustauscher 7 gefördert, und es gelangt dann über die Leitung 6 in die Extraktionskolonne 5.

Die vanillinhaltige $NaHSO_3$-, $KHSO_3$-, $Na_2SO_3$- oder $K_2SO_3$- Lösung wird dem Gaswäscher 13 über die Leitung 17 entnommen und in der Mischstrecke 18 mit verdünnter Schwefelsäure bis zu einem pH-Wert von 2 bis 4 angesäuert. Gleichzeitig wird die angesäuerte Lösung in der Mischstrecke 18 auf eine Temperatur von ca. 60°C erwärmt. Die erwärmte Lösung gelangt über die Leitung 19 in das Absetzgefäß 20, an dessen Boden sich das kristalline Vanillin abscheidet, welches diskontinuierlich über die Leitung 21 entnommen und anschließend getrocknet wird. Im Absetzgefäß 20 und in der Mischstrecke 18 wird durch das Ansäuern der vanillinhaltigen Lösung Schwefeldioxid frei, das über die Leitungen 24 und 28 abgeführt und zur Herstellung der Sulfit-Lauge verwendet wird. Die zum Ansäuern verwendete Schwefelsäure gelangt aus dem Vorratstank 23 über die Leitung 22 in die Mischstrecke 18.

Beim Ansäuern der vanillinhaltigen Hydrogensulfit- oder Sulfit-Lösung wird auch Kohlendioxid freigesetzt, das nicht vom freigesetzten Schwefeldioxid abgetrennt werden muß.

**Ausführungsbeispiel:**

Eine oxidierte Sulfit-Ablauge mit einem pH-Wert von 5,6 und einem Vanillingehalt von 6 g/kg Ablauge wurde bei kontinuierlicher Verfahrensführung 5 Stunden mit Kohlendioxid extrahiert. Der Extraktionskolonne 5 wurden pro Stunde 0,95 kg oxidierte Sulfit-Ablauge zugeführt, die mit 4,65 kg $CO_2$/Stunde bei 100 bar und 27°C extrahiert wurden. Die vanillinhaltige Kohlendioxidphase wurde bei 100 bar und 27°C durch eine Fritte in 400 ml einer $Na_2SO_3$- Lösung eingeleitet, die 75 g $Na_2SO_3$/l Wasser enthielt. Nach fünf Stunden wurde die gesamte vanillinhaltige $Na_2SO_3$-Lösung mit Schwefelsäure auf einen pH-Wert von 3,5 angesäuert und auf eine Temperatur von 50°C erwärmt. Dabei kristallisierten 26 g Vanillin aus, das einen Schmelzpunkt von 74 bis 78°C und eine Reinheit von 91,3 % hatte. Die Vanillinausbeute betrug 91 %, und das Vanillin hatte folgende

Zusammensetzung:

| | |
|---|---|
| Vanillin | 91,32 % |
| Guajacol | 0,02 % |
| Acetoguajacol | 8,2 % |
| Serinaldehyd | 0,08 % |

Für die Extraktion des Vanillins werden je nach Zusammensetzung der oxidierten Sulfit-Ablauge 0,5 bis 10 kg $CO_2$/kg Ablauge benötigt. Das Vanillin-$HSO_3$-Addukt ist sehr gut wasserlöslich und kristallisiert auch in einer konzentrierten Hydrogensulfit- oder Sulfit-Lösung nicht aus. Das Addukt kann daher direkt durch das Ansäuern der vanillinhaltigen Hydrogensulfit- oder Sulfit-Lösung gewonnen werden.

Die zur Durchführung des erfindungsgemäßen Verfahrens verwendeten Hydrogensulfite und Sulfite haben bei 30°C folgende Löslichkeit, wobei die Hydrogensulfite als Pyrosulfite eingesetzt werden, da die Hydrogensulfite in fester Form nicht beständig sind.

| | |
|---|---|
| 1,1 Mol $Na_2SO_3 \cdot 7H_2O$/l | = 249 g $Na_2SO_3 \cdot 7H_2O$/l |
| 2,1 Mol $Na_2S_2O_5$/l | = 406 g $Na_2S_2O_5$/l |
| 3,3 Mol $K_2SO_3$/l | = 518 g $K_2SO_3$/l |
| 1,6 Mol $K_2S_2O_5$/l | = 344 g $K_2S_2O_5$/l |

Um zu vermeiden, daß beim Ansäuern der vanillinhaltigen Hydrogensulfit- und Sulfit-Lösungen mit Schwefelsäure unlösliche Sulfate anfallen, die das Vanillin verunreinigen, kann beim Ansäuern nicht immer mit gesättigten Hydrogensulfit- und Sulfit-Lösungen gearbeitet werden, denn die Sulfate haben eine andere Löslichkeit als die Hydrogensulfite und Sulfite, und außerdem sind die Löslichkeiten temperaturabhängig. Daher wird in Abhängigkeit von der Temperatur, die beim Ansäuern herrscht, nur mit einer maximalen Hydrogensulfit- und Sulfitkonzentration in der wässrigen Lösung gearbeitet. Diese maximale Konzentration beträgt bei 30°C

248 g $Na_2SO_3 \cdot 7H_2O$/l
406 g $Na_2S_2O_5$/l
117 g $K_2SO_3$/l
164 $K_2S_2O_5$/l.

**Patentansprüche**

1. Verfahren zur Gewinnung von Vanillin durch Extraktion eines vanillinhaltigen, wasserhaltigen, flüssigen Stoffgemisches mit Kohlendioxid bei einer Temperatur von 0 bis 110°C sowie einem Druck von 30 bis 400 bar und anschließende Abscheidung des Vanillins aus dem vanillinhaltigen Kohlendioxid, dadurch gekennzeichnet, daß das vanillinhaltige Kohlendioxid bei der Extraktionstemperatur und dem Extraktionsdruck durch eine wässrige $NaHSO_3$-, $KHSO_3$-, $Na_2SO_3$- oder $K_2SO_3$-Lösung geleitet, daß die vanillinhaltige $NaHSO_3$-, $KHSO_3$-, $Na_2SO_3$- oder $K_2SO_3$-Lösung anschließend mit Schwefelsäure bis zu einem pH-Wert von 2 bis 4 angesäuert und daß das vanillinfreie Kohlendioxid in die Extraktionsstufe zurückgeführt wird.

2. Verfahren nach Anspruch 1, dadurch gekennzeichnet, daß das vanillinhaltige Kohlendioxid in der wässrigen $NaHSO_3$-, $KHSO_3$-, $Na_2SO_3$- oder $K_2SO_3$-Lösung eine Verweilzeit von 0,1 bis 3 Minuten hat.

3. Verfahren nach den Ansprüchen 1 bis 2, dadurch gekennzeichnet, daß die vanillinhaltige $NaHSO_3$-, $KHSO_3$-, $Na_2SO_3$- oder $K_2SO_3$-Lösung während oder nach dem Ansäuern mit Schwefelsäure auf eine Temperatur von 50 bis 90°C erhitzt wird.

4. Verfahren nach den Ansprüchen 1 bis 3, dadurch gekennzeichnet, daß als vanillinhaltiges, wasserhaltiges, flüssiges Stoffgemisch die oxidierte Ablauge des Sulfit-Holzaufschlusses verwendet wird.

**Claims**

1. A process for obtaining vanillin by extraction of a vanillin-containing, water-containing liquid mixture of substances with carbon dioxide at a temperature of 0 to 110°C and a pressure of 30 to 400 bar, followed by separation of the vanillin from the vanillin-containing carbon dioxide, characterized in that the vanillin-containing carbon dioxide is passed at the extraction temperature and the extraction pressure through an aqueous solution of $NaHSO_3$, $KHSO_3$, $Na_2SO_3$ or $K_2SO_3$; the vanillin-containing solution of $NaHSO_3$, $KHSO_3$, $Na_2SO_3$ or $K_2SO_3$ is then acidified with sulphuric acid to a pH of 2 to 4; and the vanillin-free carbon dioxide is returned to the extraction stage.

2. A process according to claim 1, characterized in that the vanillin-containing carbon dioxide has a dwell

time of 0.1 to 3 minutes in the aqueous solution of $NaHSO_3$, $KHSO_3$, $Na_2SO_3$ or $K_2SO_3$.

3. A process according to claims 1 and 2, characterized in that the vanillin-containing solution of $NaHSO_3$, $KHSO_3$. $Na_2SO_3$ or $K_2SO_3$ is heated during or after acidification with sulphuric acid to a temperature of 50 to 90°C.

4. A process according to claims 1 to 3, characterized in that the vanillin-containing, water-containing liquid mixture of substances used is the oxidized sulphite waste liquor of wood pulping.

**Revendications**

1. Procédé pour l'obtention de vanilline par extraction d'un mélange de matières liquide contenant de la vanilline et de l'eau, avec le dioxyde de carbone à une température de 0 à 110°C et sous une pression de 30 à 400 bars et séparation subséquente de la vanilline à partir du dioxyde de carbone contenant la vanilline, caractérisé en ce que le dioxyde de carbone contenant la vanilline est conduit, à la température d'extraction et la pression d'extraction, à travers une solution aqueuse de $NaHSO_3$, $KHSO_3$, $Na_2SO_3$ ou $K_2SO_3$, que la solution de $NaHSO_3$, $KHSO_3$, $Na_2SO_3$ ou $K_2SO_3$ est acidifiée ensuite avec l'acide sulfurique jusqu'à un pH de 2 à 4 et que le dioxyde de carbone exempt de vanilline est recyclé dans l'étape d'extraction.

2. Procédé selon la revendication 1, caractérisé en ce que le dioxyde de carbone contenant la vanilline séjourne dans la solution aqueuse de $NaHSO_3$, $KHSO_3$, $Na_2SO_3$ ou $K_2SO_3$ pendant 0,1 à 3 minutes.

3. Procédé selon les revendications 1 à 2, caractérisé en ce que la solution de $NaHSO_3$, $KHSO_3$, $Na_2SO_3$ ou $K_2SO_3$ contenant de la vanilline est chauffée, pendant ou après l'acidification avec l'acide sulfurique, à une température de 50 à 90°C.

4. Procédé selon les revendications 1 à 3, caractérisé en ce que comme mélange de matières liquide contenant de la vanilline et de l'eau, on utilise la lessive résiduaire oxydée de l'étape de préparation sulfitique du bois.

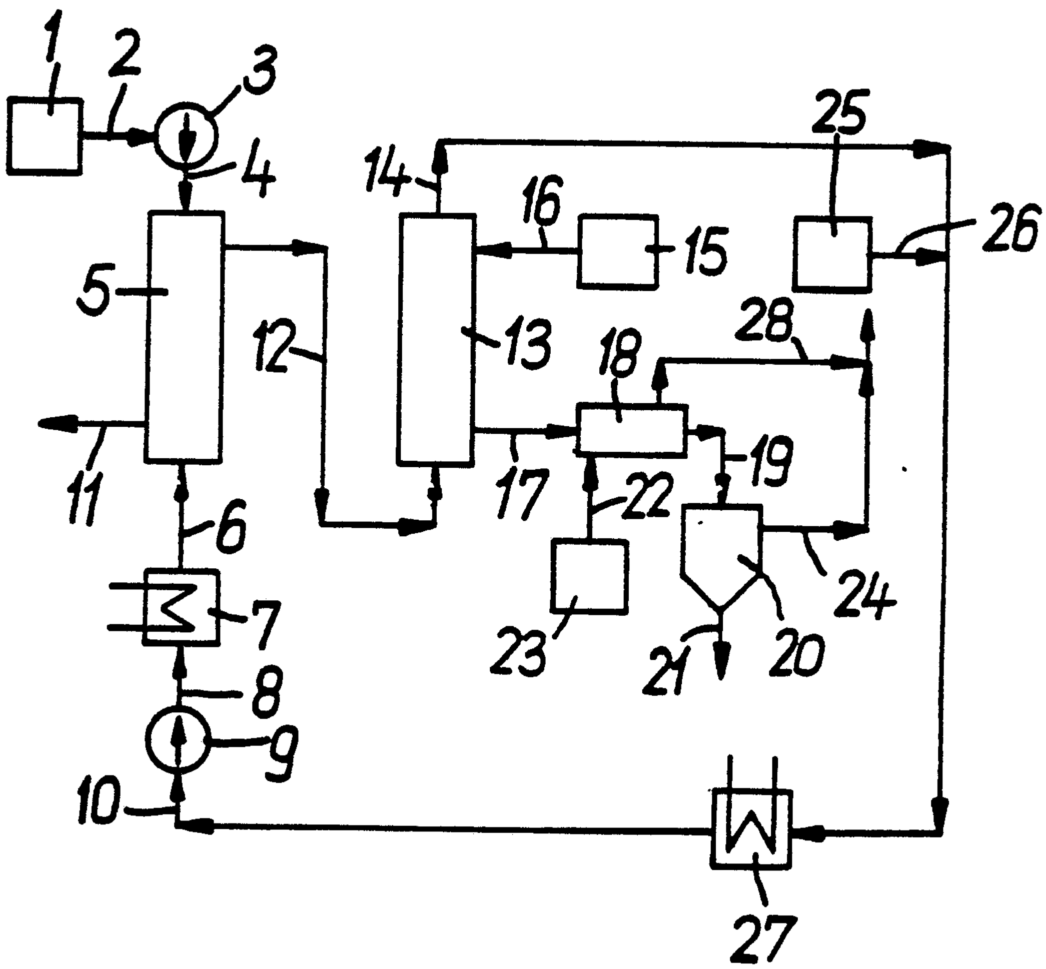
1
2
3
4
5
6
7
8
9
10
11
12
13
14
15
16
17
18
19
20
21
22
23
24
25
26
27
28